# EUROPEAN PATENT APPLICATION

(11) **EP 2 594 254 A1**
(43) Date of publication of application: **22.05.2013**
(21) Application number: 11806602.6
(22) Date of filing: 21.06.2011
(51) Int. Cl.: A61K 8/55, A61K 8/02, A61K 8/34, A61K 8/92, A61K 9/06, A61K 47/06, A61K 47/24, A61K 47/34, A61Q 19/00

(54) **OIL-BASED GEL-LIKE COMPOSITION**

(30) Priority: 16.07.2010 JP 2010161255
(71) Applicant: Daicel Chemical Industries, Ltd., Kita-ku Osaka-shi Osaka 530-0001 (JP); Nihon University, Tokyo 102-8275 (JP)
(72) Inventor: HASHIZAKI, Kaname, Tokyo 102-8275 (JP); SAITO, Yoshihiro, Tokyo 102-8275 (JP); TAGUCHI, Hiroyuki, Tokyo 102-8275 (JP); SAKANISHI, Yuichi, Ohtake-shi Hiroshima 739-0695 (JP)
(74) Representative: Grünecker, Kinkeldey, Stockmair & Schwanhäusser
(86) International application number: PCT/JP2011/064146
(87) International publication number: WO 2012/008271

(57) **Abstract**

There is provided a gel-forming agent which is easily prepared and has all of high safety in the living body and the environment, good gel-forming capability, excellent use feeling, and good handleability.

The oil-based gel-like composition of the present invention comprises 1 to 30% by weight of a gel-forming agent and 70 to 99% by weight of an oil-phase component, the gel-forming agent being obtained by blending polyglycerin having a degree of polymerization of 3 to 20 in an amount of [6.8 Ln(X) + 17] parts by weight to 27 Ln(X) parts by weight with 100 parts by weight of lecithin, where X denotes the degree of polymerization of the polyglycerin. The degree of polymerization of the polyglycerin is preferably from 3 to 10. The zero-shear viscosity determined by the viscosity and viscoelasticity measurement is preferably 50 Pa·s or more.

## Description

### Technical Field

The present invention relates to a gel-forming agent and an oil-based gel-like composition comprising the gel-forming agent and an oil-phase component.

### Background Art

The gel-forming agent for solidifying various oil-phase components such as animal and vegetable oils, mineral oils, hydrocarbons, and fatty acid esters by thickening them or forming a gel thereof is widely used in various fields such as cosmetics, drugs, food, coatings, ink, and lubricating oil. The performance generally required for the gel-forming agent includes the ability of causing gelation of a target oil-phase component by adding a small amount thereof and the ability of providing a gel which is stable over a long period of time. It is also required that, depending on applications, the gel-forming agent have high safety in the human body and the environment, produce a thixotropic gel, and provide a gel having good feel.

Conventionally known gel-forming agents include low-molecular gel-forming agents (such as 1,2,3,4-dibenzylidene-D-sorbitol, 12-hydroxy stearic acid, and amino acid derivatives) and polymeric gel-forming agents (such as polyacrylic acid derivatives and dextrin derivatives). The low-molecular gel-forming agents self-assemble in an oil-phase component to form a huge network structure to thereby immobilize the oil-phase component to form a gel; on the other hand, the polymeric gel-forming agents are intricately entangled to form a network structure to thereby cause the gelation of the oil-phase component.

On the other hand, although only a few reports are available, the gelation of an oil-phase component with reverse worm-like micelles is also reported (Non Patent Literatures 1 to 6). The reverse worm-like micelle is a type of a self assembly which is formed by a surfactant and is known to cause gelation because the reverse worm-like micelle forms a network structure in the oil-phase component. Since the reverse worm-like micelle has a hydrophilic environment in the inner part thereof, it can contain a water-soluble drug, enzyme, and the like therein and has a feature not available in the gel-forming agents as described above.

As a typical system which forms the reverse worm-like micelle, a three-component mixture system comprising lecithin/water/various oil-phase components is reported (Non Patent Literature 1). Further, ethylene glycol, formamide, glycerin, bile salt (Non Patent Literature 3), urea (Non Patent Literature 4), sucrose fatty acid ester (Non Patent Literature 5), and D-ribose and D-deoxyribose (Non Patent Literature 6) are reported as the alternative materials of water. Generally, lecithin forms reverse spherical micelles or reverse elliptical micelles in the oil-phase component, but if a small amount of water or the like is added thereto, it is probably hydrogen-bonded to a phosphate group of lecithin to reduce the interface curvature of the molecular assembly to thereby cause the growth of the reverse worm-like micelles.

Further, as a method for causing gelation of the oil-phase component in addition to the methods as described above, the gelation of the oil-phase component by an emulsion is reported (Patent Literature 1). That is, it is a gel emulsion prepared by using one surfactant including lecithin and sucrose fatty acid esters or a combination of two or more thereof and thereto adding a higher alcohol, glycerin, and an oil-phase component.

### Citation List

### Patent Literature

Patent Literature 1: Japanese Unexamined Patent Application (JP-A) No. H5-4911

### Non Patent Literature

Non Patent Literature 1: P. L. Luisi et al., Colloid & Polymer Science, vol. 268, p. 356 (1990)
Non Patent Literature 2: Yu. A. Shchipunov, Colloids and Surfaces A, vol.183-185, p. 541 (2001)
Non Patent Literature 3: S. H. Tung et al. Journal of the American Chemical Society, vol. 128, p. 5751 (2006)
Non Patent Literature 4: K. Hashizaki et al., Colloid & Polymer Science, vol. 287, p. 927 (2009)
Non Patent Literature 5: K. Hashizaki et al., Colloid & Polymer Science, vol. 287, p. 1099 (2009)
Non Patent Literature 6: K. Hashizaki et al., Chemistry Letters, vol. 38, p. 1036 (2009)

### Summary of Invention

### Technical Problem

Although 1,2,3,4-dibenzylidene-D-sorbitol as a low-molecular gel-forming agent as described above is an excellent compound which can cause gelation of various types of oil-phase components, it has a safety problem in that it is decomposed to produce benzaldehyde and has not been put to practical use. Although 12-hydroxy stearic acid is commercially available as a gel-forming agent of waste tempura oil, it has poor thixotropy. Further, since an amino acid derivative as a gel-forming agent is poorly soluble in an oil-phase component, complicated operations such as heating at high temperatures and a long-time stirring are required for dissolving it. Moreover, there is a problem also in that such operations may cause the change of quality of other components to be blended with the gel. On the other hand, a dextrin derivative as a polymeric gel-forming agent needs to be added in a high concentration, and in addition it produces a "feeling of stickiness" peculiar to polymers and provides a poor use feeling. A polyacrylic acid derivative shows good thickening and gel formation by a small amount of addition, but when it is used for the skin, it produces a "feeling of stickiness" peculiar to polymers and provides a poor use feeling.

Next, conventional reverse worm-like micelles have a problem in that a representative reverse worm-like micelle comprising lecithin/water/various oil-phase components cannot incorporate a drug or the like which is susceptible to hydrolysis because water is included in the components. The conventional reverse worm-like micelles also have a problem in that when glycerin which is liquid at normal temperature is used as an alternative material of water, gel-forming capability is insufficient, and when ethylene glycol and formamide which are similarly liquid at normal temperature are used as alternative materials of water, they have strong stimulativeness to the skin, eye, mucous membrane, and the like and cannot be applied to the human body. The conventional reverse worm-like micelles also have a problem in that when bile salt, urea, sucrose fatty acid ester, D-ribose, and the like which are solid at normal temperature are used as alternative materials of water, a preparation method will be complicated compared with the case where a liquid is used. Therefore, a gel-forming agent which is easily prepared and has all of high safety in the living body and the environment, good gel-forming capability, excellent use feeling, and good handleability has not been obtained until now.

Further, Patent Literature 1 discloses a gel emulsion prepared by using one surfactant including lecithin and sucrose fatty acid esters or a combination of two or more thereof and thereto adding a higher alcohol, glycerin, and an oil-phase component. Since this gel has lower elasticity than the gel comprising the gel-forming agent and the reverse worm-like micelle as described above, it easily drips and has poor handleability, and in addition, it has a problem that when higher alcohol and/or glycerin is not added, the effect cannot be obtained.

Therefore, an object of the present invention is to provide a gel-forming agent which is easily prepared and has all of high safety in the living body and the environment, good gel-forming capability, excellent use feeling, and good handleability.
Another object of the present invention is to provide an oil-based gel-like composition excellent in gel stability comprising a gel-forming agent having the above excellent characteristics and an oil-phase component.

### Solution to Problem

As described above, when a gel-forming agent and a thickened gel-like composition are used in various fields such as cosmetics, drugs, food, coatings, ink, and lubricating oil, the performance required includes the ability of causing gelation of a target oil-phase component by adding a small amount thereof and the ability of providing a gel which is stable over a long period of time. It is also required that, depending on applications, the gel-forming agent and the thickened gel-like composition have high safety in the human body and the environment, produce a thixotropic gel, and provide a gel having good feel. However, it has been impossible for prior art to provide a sufficient gel-forming agent having all of the performance.

Now, as a result of intensive studies to solve the above problems, the present inventors have succeeded in obtaining a gel-forming agent and a thickened gel-like composition comprising reverse worm-like micelles using a three-component mixture system of lecithin/polyglycerin/oil-phase component.

The lecithin used in the present invention is an amphoteric phospholipid having two alkyl chains, which is used as a food emulsifier for emulsifying dairy products and reducing the viscosity of chocolate, is widely used for pharmaceutical preparations and the like, and has high safety in the living body and the environment. On the other hand, polyglycerin is used as a moisturizer of cosmetics and a food emulsifier with its strong hydrogen bonding ability and high safety. In the present invention, it has been found that when a three-component mixture system of lecithin/polyglycerin/oil-phase component is used for the preparation of a gel-forming agent which can form a reverse worm-like micelle, each polyglycerin has a blending range suitable for gelation depending on the degree of polymerization of the polyglycerin, and more specifically, there is a correlation between a suitable blending amount of polyglycerin and a value of the natural logarithm of the degree of polymerization of the polyglycerin.

Specifically, the present invention provides an oil-based gel-like composition comprising 1 to 30% by weight of a gel-forming agent and 70 to 99% by weight of an oil-phase component, the gel-forming agent being obtained by blending a polyglycerin having a degree of polymerization of 3 to 20 in an amount of [6.8 Ln(X) + 17] parts by weight to 27 Ln(X) parts by weight with 100 parts by weight of lecithin, where X denotes the degree of polymerization of the polyglycerin.

In the oil-based gel-like composition, the degree of polymerization of the polyglycerin is more preferably from 3 to 10.

Further, the zero-shear viscosity determined by the viscosity and viscoelasticity measurement is preferably 50 Pa·s or more.

The present invention also provides a gel-forming agent obtained by blending a polyglycerin having a degree of polymerization of 3 to 20 in an amount of [6.8 Ln(X) + 17] parts by weight to 27 Ln(X) parts by weight with 100 parts by weight of lecithin, where X denotes the degree of polymerization of the polyglycerin.

In the gel-forming agent, the degree of polymerization of the polyglycerin is more preferably from 3 to 10.

Note that the oil-based gel-like composition according to the present invention means a gel-like composition which does not contain water or contains only a trace amount (for example, 1% by weight or less, preferably 0.2% by weight or less) of water.

### Advantageous Effects of Invention

Since the gel-forming agent of the present invention is obtained by blending lecithin with a polyglycerin having a specific degree of polymerization in a specific proportion, it is easily prepared and has all of high safety in the living body and the environment, good gel-forming capability, excellent use feeling, and good handleability. It is excellent also in transparency.
Since the oil-based gel-like composition of the present invention comprises the above excellent gel-forming agent and oil-phase component in a specific proportion, it has high safety in the living body and the environment, is excellent in use feeling, and is excellent in gel stability. It is excellent also in transparency.

### Brief Description of Drawings

[Figure 1] Figure 1 is a view showing the results of rheology measurement of a lecithin/tetraglycerin/n-decane system.
[Figure 2] Figure 2 is a view showing the results of rheology measurement of a lecithin/glycerin/n-decane system.

### Description of Embodiments

### [Lecithin]

Lecithin is a lipid product essentially comprising phosphatidylcholine, which is widely distributed in the living body such as natural animals, plants, and microorganisms, and it is known to be contained in a large amount in liver, yolk, soybeans, yeast, and the like. Representative lecithin includes yolk lecithin and soybean lecithin. Lecithin can be used alone or in combination of two or more thereof. A lecithin having a phosphatidylcholine content of about 55 to 99% by weight is preferred. A lecithin having a phosphatidylcholine content in this range easily forms a cream-like product, has proper consistency, does not flow and drop when it is applied to the skin, and has good use feeling. Although natural lecithin is only the L-α-form, other forms can also be used. Since natural lecithin is easily oxidized and unstable, it may be hydrogenated by a known method when it is used. Such a hydrogenated lecithin can also be included in "lecithin" in the present invention.

Phosphatidylcholine means an ester obtained by allowing glycerol (glycerin) to react with at least one unsaturated fatty acid and phosphoric acid, and a proton of the phosphoric acid is replaced by choline as an amine functional group. In the present invention, "phosphatidylcholine" includes a phosphatidylcholine in which an unsaturated bond is hydrogenated.

In the present invention, phosphatidylcholine is particularly defined according to the following general formula (I). In the formula, R₁ and R₂ each independently represent an aliphatic hydrocarbon group derived from (corresponding to) a saturated or unsaturated fatty acid having 4 to 24 carbon atoms (that is, a saturated or unsaturated aliphatic hydrocarbon group having 3 to 23 carbon atoms), and the R₁ and R₂ may be linear or branched and may be replaced by one or more hydroxyl functional groups and/or amine functional groups; and X represents a choline residue. One of the compounds represented by formula (I) or a mixture of two or more thereof may be considered as phosphatidylcholine.

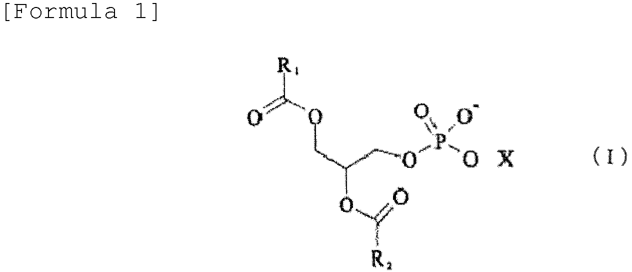

In one of the embodiments of the present invention, a fatty acid corresponding to R₁ and R₂ (R₁COOH, R₂COOH) is selected, for example, from butyric acid, caproic acid, caprylic acid, capric acid, caproleic acid, lauric acid, lauroleic acid, myristic acid, myristoleic acid, palmitic acid, palmitoleic acid, stearic acid, oleic acid, linoleic acid, linolenic acid, arachidic acid, isostearic acid, dihydroxystearic acid, and recinoleic acid.

A non-hydrogenated phosphatidylcholine (PC) which is suitable for the practice of the composition of the present invention may be derived from "nature", or may be made "synthetically".

"Natural" PC may be obtained by extraction from an animal source or a vegetable source, for example, a soybean, a sunflower, or an egg. The non-hydrogenated phosphatidylcholine obtained from a natural product, for example, from a soybean generally contains palmitic acid, stearic acid, palmitoleic acid, oleic acid, linoleic acid, and linolenic acid, and probably a C20 to C22 fatty acid as a fatty acid for esterifying glycerol.

### [Polyglycerin]

The polyglycerin used in the present invention is produced by various methods. Examples include (1) a method of addition-polymerizing epichlorohydrin with glycerin, followed by dehydrochlorination-cyclization under an alkali condition, followed by ring opening with dilute sulfuric acid, wherein these operations are repeated until a target degree of polymerization is reached; (2) a method of adding glycidol to glycerin; (3) a method, in connection with the method (2), of adding glycidol whose hydroxy group is protected to glycerin, followed by deprotection, wherein these operations are repeated until any given degree of polymerization is reached; (4) a method of subjecting glycerin to thermal condensation in the presence of an alkali; and (5) a method of adding an allyl halide to glycerin, followed by epoxidation, followed by ring opening with water, wherein these operations are repeated until any given degree of polymerization is reached; and the most preferred production method is the method of (2), which can be suitably used for producing the gel-forming agent of the oil-based gel-like composition.

The degree of polymerization of the polyglycerin to be used is preferably 3 to 20. If it is less than 3, a stable gel cannot be obtained, but if it is larger than 20, the resulting composition will be emulsified, preventing a transparent gel from being obtained or preventing a gel itself from being obtained. In particular, a polyglycerin having a degree of polymerization of 3 to 10 is preferably used. The polyglycerin may be used alone or in combination of a plurality thereof having a different degree of polymerization.

### [Gel-forming Agent]

The gel-forming agent of the present invention is obtained by blending a polyglycerin having a degree of polymerization of 3 to 20 in an amount of [6.8 Ln(X) + 17] parts by weight to 27 Ln(X) parts by weight with 100 parts by weight of lecithin, where X denotes the degree of polymerization of the polyglycerin. The above "Ln" denotes the natural logarithm "logₑ".

More specifically, when the degree of polymerization of polyglycerin is 3, 24 to 30 parts by weight of the polyglycerin is blended with 100 parts by weight of lecithin; when the degree of polymerization of polyglycerin is 4, 27 to 38 parts by weight of the polyglycerin is blended with 100 parts by weight of lecithin; when the degree of polymerization of polyglycerin is 5, 28 to 43 parts by weight of the polyglycerin is blended with 100 parts by weight of lecithin; when the degree of polymerization of polyglycerin is 6, 29 to 49 parts by weight of the polyglycerin is blended with 100 parts by weight of lecithin; when the degree of polymerization of polyglycerin is 10, 33 to 62 parts by weight of the polyglycerin is blended with 100 parts by weight of lecithin; and when the degree of polymerization of polyglycerin is 20, 37 to 81 parts by weight of the polyglycerin is blended with 100 parts by weight of lecithin. When a polyglycerin each having a degree of polymerization of 3 to 20 is mixed and the mixture has an average degree of polymerization of Xa, the mixed glycerin in an amount in the range of [6.8 Ln(Xa) + 17] parts by weight to 27 Ln(Xa) parts by weight may be blended with 100 parts by weight of lecithin.

As described above, since a lecithin having a phosphatidylcholine content of 55 to 99% by weight is preferred, a gel-forming agent is preferred in which a polyglycerin having a degree of polymerization of 3 to 20 in an amount of [6.8 Ln(X) + 17] parts by weight to 27 Ln(X) parts by weight is blended with 55 to 99 parts by weight (for example, 75 parts by weight) of phosphatidylcholine, where X denotes the degree of polymerization of the polyglycerin.

The gel-forming agent can be included in an amount of 1 to 30% by weight relative to the whole oil-based gel-like composition, wherein the gel-forming agent is preferably included in an amount of 5 to 20% by weight, particularly preferably included in an amount of 10 to 15% by weight.

The content of lecithin in the whole oil-based gel-like composition can be calculated from the above, and it is preferably in the range of 2 to 27% by weight, particularly preferably in the range of 6 to 13% by weight. If the content of lecithin is low, the gelation tends to be poor, and a stable oil-based gel-like composition cannot be obtained. Further, if the content is too high, gel-forming strength and moisturizing/water-retention effect will be saturated; therefore, there is no merit in using a large amount of lecithin, and it is not economical. Therefore, the content in the range as described above is preferred. Note that the content of phosphatidylcholine in the whole oil-based gel-like composition is, for example, 1.5 to 26% by weight, preferably 4 to 12% by weight.

If the content of the gel-forming agent (the total amount of lecithin and polyglycerin having a degree of polymerization of 2 to 10) relative to the whole oil-based gel-like composition is less than 1% by weight, the gelation will be poor, and a stable oil-based gel-like composition cannot be obtained. Further, if the content of the gel-forming agent relative to the whole oil-based gel-like composition is too high, gel-forming strength and moisturizing/water-retention effect will be saturated; therefore, there is no merit in using a large amount of gel-forming agent, and it is not economical. Therefore, the content in the range as described above is preferred.

The content of polyglycerin having a degree of polymerization of 3 to 20 in the whole oil-based gel-like composition can also be calculated from the above, and it is preferably in the range of 0.2 to 13% by weight, particularly preferably in the range of 1 to 6% by weight. If the content of polyglycerin having a degree of polymerization of 3 to 20 is low, a stable oil-based gel-like composition cannot be obtained; and if the content of polyglycerin having a degree of polymerization of 3 to 20 is too high, gel-forming strength and moisturizing/water-retention effect will be saturated, and therefore, there is no merit in using a large amount of polyglycerin, and it is not economical.

### [Oil-phase Component]

The oil-phase component used in the present invention essentially comprises only a polar oil, a mixture of a polar oil and a nonpolar oil, or a nonpolar oil. Examples of the nonpolar oil include hydrocarbons such as squalane, vaseline, and liquid paraffin, and open-chain or cyclic silicone oils; and examples of the polar oil include oils and fats such as olive oil, wax such as lanolin, esters such as isopropyl myristate, decyl oleate, and glycerin tri-2-ethyl hexanoate [an ester of a fatty acid having 8 or more carbon atoms (preferably, having 8 to 25 carbon atoms) with an alcohol and the like], higher fatty acids such as oleic acid and lauric acid [a fatty acid having 12 or more carbon atoms (preferably, having 12 to 25 carbon atoms) and the like], and higher alcohols such as cetanol which are solid at normal temperature [an alcohol having 12 or more carbon atoms (preferably, having 12 to 25 carbon atoms) and the like]. These oil-phase components are each blended alone or in combination in a content in the range from 70 to 99% by weight relative to the total amount of the oil-based gel-like composition. If the content of the oil-phase component is lower than 70% by weight, the amount of the gel-forming agent will be too much; and if the content exceeds 99% by weight, the stability of gel will be reduced, and this content is not economical; therefore, the range as described above is preferred. The content of the oil-phase component is preferably 80 to 95% by weight, more preferably 85 to 90% by weight, relative to the total amount of the oil-based gel-like composition.

### [Others]

Lecithin is effective as a cosmetic in that lecithin itself spreads to all the corners of the keratin of the skin to soften the aged keratin, and in order to further enhance the effect as a cosmetic for the skin, it can be mixed with components such as vitamin B, vitamin E, and various types of fragrance. As the additive component, ascorbic acid is particularly effective. Ascorbic acid has a pH value of about 2, and when it is added, the pH value of a cosmetic will be reduced and a keratolysis effect is exhibited to remove the old keratin. In order to blend ascorbic acid more stably, ascorbic acid derivatives such as ascorbyl palmitate can be used. Further, if a component having an antimicrobial activity such as hinokitiol, fucoidan, and salicylic acid is added, it is also possible to cope with fungus, bacteria, and the like which are present in keratin. Further, if a vegetable antiphlogistic/moisturizing component such as glycyrrhizic acid is blended, a killing/moisturizing action to a hardened keratin in a lacerated and inflamed state can be expected. Furthermore, in order to prevent stickiness, a powder such as silica, silicon powder, and an alkyl acrylate copolymer can also be added.

In the oil-based gel-like composition of the present invention, a component used for common cosmetics can be mixed in addition to the components as described above. Examples include fragrance, dyes, preservatives, antioxidants, antiinflammatory agents, ultraviolet absorbers, ultraviolet reflecting agents, and pH adjusters, and optionally include various pharmaceutically active components such as hyaluronic acid, allantoin, vitamins, amino acid, and placenta extract; these may be suitably blended alone or in combination.

The content of the components other than the gel-forming agent and the oil-phase component in the oil-based gel-like composition of the present invention is generally 29% by weight or less (for example, 0.1 to 29% by weight), preferably 20% by weight or less (for example, 0.1 to 20% by weight), more preferably 10% by weight or less (for example, 0.1 to 10% by weight).

The oil-based gel-like composition obtained in the present invention is stable over a long period of time, for example, for three months or more. Further, from the recognition of having proper elasticity in the rheology measurement, it can be judged that the composition hardly drips and has good handleability. Furthermore, since the composition is thixotropic, it has good elongation, for example, when it is applied to the skin or the like.

The zero-shear viscosity determined by the viscosity and viscoelasticity measurement of the oil-based gel-like composition of the present invention is preferably 50 Pa·s or more, particularly preferably 100 Pa·s or more, in terms of gel stability, and feel, use feeling, handleability, and the like of the gel. The zero-shear viscosity has no particular upper limit, and it differs depending on applications; it is for example 2000 Pa.s, preferably 1000 Pa.s.

### Examples

Hereinafter, the present invention will be described more specifically with reference to Examples, but the present invention is not limited to these Examples.

### Examples 1 to 11, Comparative Examples 1 to 26

Lecithin and polyglycerin (or glycerin) were mixed with n-decane (oil-phase component) in proportions shown in Tables 2 to 7 to prepare oil-based gel-like compositions. The obtained oil-based gel-like compositions were evaluated for the viscosity increase and gel formation by performing rheology measurement and the transparency of each composition. The results are shown in Tables 2 to 7. The numerical values in the Tables represent the blending ratio (% by weight) of each component. The reagents, preparation methods, and evaluation methods which were used are shown below. Note that, in the Tables, poly (20) glycerin represents a polyglycerin having a degree of polymerization of 20, and poly (40) glycerin represents a polyglycerin having a degree of polymerization of 40.

### <Reagents>

Soybean lecithin was used as the lecithin. A soybean lecithin available from Avanti Polar Lipids and Inc. was used. Special grade articles of glycerin and n-decane available from Kanto Chemical Co., Inc. were used as they were. Various types of polyglycerin manufactured by Daicel Corporation were used as they were.

### <Preparation Method>

Required amounts of lecithin, polyglycerin or glycerin, and n-decane were sealed in a bottle and stirred overnight with a magnetic stirrer. Subsequently, the resulting mixture was allowed to stand for several days in a temperature controlled bath at 25°C to allow the equilibrium to be reached, thus obtaining a sample.

### <Evaluations>

The compositions obtained in Examples and Comparative Examples were evaluated by the following methods.

### (1) Rheology Measurement

The measurement was performed using a viscosity and viscoelasticity measuring instrument (RheoStress600, manufactured by HAAKE) equipped with a cone plate sensor (a sensor used had a diameter of 60 mm and a cone angle of 1°, or a diameter of 35 mm and a cone angle of 1°, 2°, or 4°), and a Peltier temperature controller. All the measurements were performed in a steady flow viscosity measurement mode under a 25°C condition, wherein the viscosity was measured by changing the shear rate from 0.001 to 100 (s⁻¹) in the unit of logarithm to obtain viscosity curves. Further, a value obtained when the torque value variation of the instrument was settled within the range of 5% and the data were stabilized was employed for each plot.

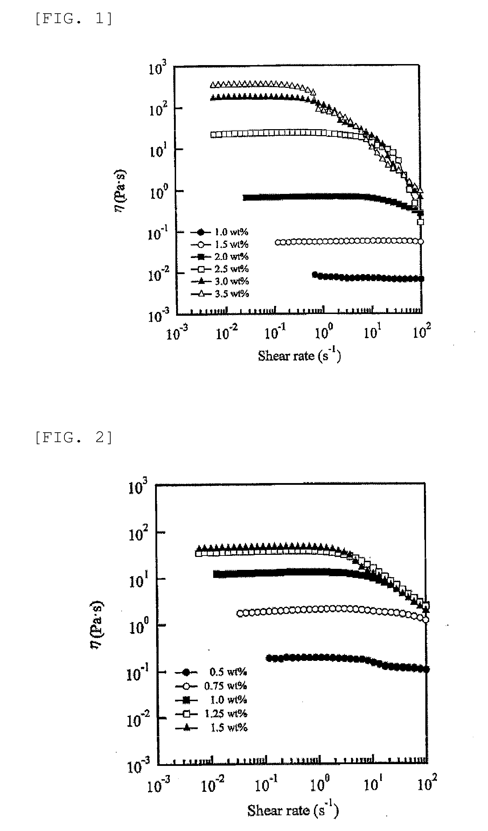

Figure 1 shows the results of rheology measurement of a lecithin/tetraglycerin/n-decane system (lecithin: 10% by weight, tetraglycerin: 1.0 to 3.5% by weight, and n-decane: the balance) (Examples 1 and 2, Comparative Examples 2 to 5). The numbers in the figure represents the concentration of tetraglycerin (% by weight). The ordinate represents the viscosity η (Pa.s), and the abscissa represents the shear rate (s⁻¹). It is found that a non-Newtonian flow is shown, because viscosity decreases as the shear rate is increased.

Figure 2 shows the results of rheology measurement of a lecithin/glycerin/n-decane system (lecithin: 10% by weight, glycerin: 0.5 to 1.5% by weight, and n-decane: the balance) (Comparative Examples 18 to 21). The numbers in the figure represents the concentration of glycerin (% by weight). The ordinate represents the viscosity η (Pa.s), and the abscissa represents the shear rate (s⁻¹). It is found that the viscosity is lower than in the case of Figure 1 (when tetraglycerin was used).

### (2) Zero-shear viscosity η0

Oil-based gel-like compositions were prepared by mixing lecithin, polyglycerin (or glycerin), and n-decane in proportions as shown in Table 1, and the obtained oil-based gel-like compositions were subjected to rheology measurement, in which the zero-shear viscosity η0 of each composition was determined from the viscosity curves obtained in the rheology measurement. That is, in a region where the shear rate is infinitely close to zero, even a non-Newtonian fluid can be approximated to Newtonian fluid, and the viscosity in the region does not vary and shows a certain value. The viscosity η at this time can be treated as the zero-shear viscosity η0. The viscosity was maintained at a certain value at a shear rate of 0.1 (s⁻¹) or less, and this value was defined as a zero-shear viscosity η0. The measured values of the zero-shear viscosity of each composition are shown in Table 1. The numbers under the columns of Lecithin, (Poly)glycerin, and n-Decane represent the content (% by weight) in the compositions. The numbers under the column of the Degree of polymerization of glycerin represent the zero-shear viscosity η0 (Pa.s) when a (poly)glycerin having each degree of polymerization of glycerin was used.

[Table 1]

**Table 1**

| Lecithin | (Poly) glycerin | n-Decane | Degree of polymerization of glycerin | | | | | |
|---|---|---|---|---|---|---|---|---|
| | | | 1 | 3 | 4 | 5 | 6 | 10 |
| 10 | 0.5 | 89.5 | 0.1881 | | | | | |
| 10 | 0.75 | 89.25 | 1.905 | | | | | |
| 10 | 1 | 89 | 12.8 | 0.03274 | 0.007071 | 0.004922 | | |
| 10 | 1.25 | 88.75 | 37.03 | | | | | |
| 10 | 1.5 | 88.5 | 44.63 | 0.2097 | 0.0561 | 001636 | 0.008665 | 0.002609 |
| 10 | 2 | 88 | | 5.475 | 0.6629 | 0.1926 | 0.1237 | 0.01557 |
| 10 | 2.5 | 87.5 | | 88.95 | 23.47 | 5.949 | 4.013 | 0.2827 |
| 10 | 3 | 87 | | 171.6 | 173.9 | 118.6 | 71.87 | 13.32 |
| 10 | 3.5 | 86.5 | | | 350.6 | 442.6 | 427.9 | 221.6 |

### (3) Evaluation of Viscosity Increase and Gel Formation

The zero-shear viscosity η0 (Pa.s) was determined in the same manner as described above for the oil-based gel-like compositions obtained in Examples 1 to 11 and Comparative Examples 1 to 26. The evaluation of viscosity increase and gel formation (thickening and gelation) was determined as follows based on the zero-shear viscosity η0 (Pa·s). The results are shown in Tables 2 to 7.
⊙: Zero-shear viscosity η0 is 100 Pa·s or more.
○: Zero-shear viscosity η0 is 50 Pa·s or more and less than 100 Pa.s.
×: Zero-shear viscosity η0 is less than 50 Pa.s.

### (4) Evaluation of Transparency

The transparency was determined as follows by visual observation for the oil-based gel-like compositions obtained in Examples 1 to 11 and Comparative Examples 1 to 26. The results are shown in Tables 2 to 7.
⊙: Transparent
○: Translucent
Δ: Cloudy
×: Two-phase separated

[Table 2]

**Table 2**

| | Example | | | | |
|---|---|---|---|---|---|
| | 1 | 2 | 3 | 4 | 5 |
| Lecithin | 10 | 10 | 10 | 10 | 10 |
| Tetraglycerin | 3 | 3.5 | | | |
| Hexaglycerin | | | 3 | 3.5 | 4 |
| n-Decane | 87 | 86.5 | 87 | 86.5 | 86 |
| Thickening and gelation | ⊙ | ⊙ | ○ | ⊙ | ⊙ |
| Transparency | ⊙ | ⊙ | ⊙ | ⊙ | ○ |

[Table 3]

**Table 3**

| | Example | | | | | |
|---|---|---|---|---|---|---|
| | 6 | 7 | 8 | 9 | 10 | 11 |
| Lecithin | 10 | 10 | 10 | 10 | 10 | 10 |
| Decaglycerin | 3.5 | 4 | 5 | 6 | | |
| Poly(20)glycerin | | | | | 4 | 5 |
| n-Decane | 86.5 | 86 | 85 | 84 | 86 | 85 |
| Thickening and gelation | ⊙ | ⊙ | ⊙ | ⊙ | ⊙ | ⊙ |
| Transparency | ⊙ | ○ | Δ | Δ | ○ | ○ |

[Table 4]

**Table 4**

| | Comparative Example | | | | | | | | |
|---|---|---|---|---|---|---|---|---|---|
| | 1 | 2 | 3 | 4 | 5 | 6 | 7 | 8 | 9 |
| Lecithin | 10 | 10 | 10 | 10 | 10 | 10 | 10 | 10 | 10 |
| Tetraglycerin | 0.5 | 1 | 1.5 | 2 | 2.5 | | | | |
| Hexaglycerin | | | | | | 0.5 | 1 | 1.5 | 2.5 |
| n-Decane | 89.5 | 89 | 88.5 | 88 | 87.5 | 89.5 | 89 | 88.5 | 87.5 |
| Thickening and gelation | × | × | × | × | × | × | × | × | × |
| Transparency | × | ⊙ | ⊙ | ⊙ | ⊙ | × | × | ⊙ | ⊙ |

[Table 5]

**Table 5**

| | Comparative Example | | |
|---|---|---|---|
| | 10 | 11 | 12 |
| Lecithin | 10 | 10 | 10 |
| Decaglycerin | 1 | 1.5 | |
| Poly(20)glycerin | | | 1 |
| n-Decane | 89 | 88.5 | 89 |
| Thickening and gelation | × | × | × |
| Transparency | × | ⊙ | × |

[Table 6]

**Table 6**

| | Comparative Example | | | | |
|---|---|---|---|---|---|
| | 13 | 14 | 15 | 16 | 17 |
| Lecithin | 10 | 10 | 10 | 10 | 10 |
| Poly(40)glycerin | 1 | 2 | 3 | 4 | 5 |
| n-Decane | 89 | 88 | 87 | 86 | 85 |
| Thickening and gelation | × | × | × | × | × |
| Transparency | ⊙ | ⊙ | × | × | × |

[Table 7]

**Table 7**

| | Comparative Example | | | | | | | | |
|---|---|---|---|---|---|---|---|---|---|
| | 18 | 19 | 20 | 21 | 22 | 23 | 24 | 25 | 26 |
| Lecithin | 10 | 10 | 10 | 10 | 10 | 10 | 10 | 10 | 10 |
| Glycerin | 0.75 | 1 | 1.25 | 1.5 | 1. 75 | 2 | 3 | 4 | 5 |
| n-Decane | 89 | 89 | 88.8 | 88.5 | 88.3 | 88 | 87 | 86 | 85 |
| Thickening and gelation | × | × | × | × | × | × | × | × | × |
| Transparency | ⊙ | ⊙ | ⊙ | ⊙ | × | × | × | × | × |

### Industrial Applicability

Since the gel-forming agent and thickened gel-like composition featured by comprising reverse worm-like micelles obtained in the present invention are extremely safe to the human body and the environment, they can be used as various products which are in a gel state at normal temperature such as cosmetics, drugs, food, detergent, deodorizers, bath fragrance, fragrance, and deodorants. Among others, they are particularly suitable for the applications of cosmetics and drugs. The cosmetics include cream, milky lotion, lotion, cleansing cream, bath cosmetics, moisturizing cosmetics, circulation promotion and massaging agents, pack cosmetics, and hair cosmetics. The drugs include ointments, molded cataplasms, gradual-release formulation substrates, percutaneous absorption preparations, drug delivery system carriers, and gels for electrophoresis.

## Claims

1. An oil-based gel-like composition comprising 1 to 30% by weight of a gel-forming agent and 70 to 99% by weight of an oil-phase component, the gel-forming agent being obtained by blending polyglycerin having a degree of polymerization of 3 to 20 in an amount of [6.8 Ln(X) + 17] parts by weight to 27 Ln(X) parts by weight with 100 parts by weight of lecithin, where X denotes the degree of polymerization of the polyglycerin.

2. The oil-based gel-like composition according to claim 1, wherein the degree of polymerization of the polyglycerin is from 3 to 10.

3. The oil-based gel-like composition according to claim 1 or 2, wherein the zero-shear viscosity determined by the viscosity and viscoelasticity measurement is 50 Pa·s or more.

4. A gel-forming agent obtained by blending polyglycerin having a degree of polymerization of 3 to 20 in an amount of [6.8 Ln(X) + 17] parts by weight to 27 Ln(X) parts by weight with 100 parts by weight of lecithin, where X denotes the degree of polymerization of the polyglycerin.

5. The gel-forming agent according to claim 4, wherein the degree of polymerization of the polyglycerin is from 3 to 10.
